# EUROPEAN PATENT SPECIFICATION

(11) **EP 3 372 247 B1**
(45) Date of publication and mention of the grant of the patent: **05.01.2022**
(21) Application number: 16862483.1
(22) Date of filing: 04.11.2016
(51) Int. Cl.: A61K 47/12, A61K 47/18, A61K 38/00, A61K 38/38, A61K 9/00, A61P 5/00, A61P 43/00

(54) **COMPOSITION FOR SKIN PERMEATION COMPRISING CATIONIC MOLECULAR TRANSPORTER AND PROTEIN**
ZUSAMMENSETZUNG ZUR HAUTPERMEATION MIT KATIONISCHEM MOLEKULAREM TRANSPORTER UND PROTEIN
COMPOSITION POUR PÉNÉTRATION CUTANÉE COMPRENANT UN TRANSPORTEUR MOLÉCULAIRE CATIONIQUE ET UNE PROTÉINE

(30) Priority: 04.11.2015 KR 20150154384
(43) Date of publication of application: 12.09.2018
(73) Proprietor: Postech Academy-Industry Foundation, Pohang-si, Gyeongsangbuk-do 37673 (KR)
(72) Inventor: CHUNG, Sung-Kee, Gyeongju-si Gyeongsangbuk-do 38176 (KR); IM, Jungkyun, Asan-si Chungcheongnam-do 31523 (KR); LEE, Woo Sirl, Daejeon 34049 (KR)
(74) Representative: dompatent von Kreisler Selting Werner - Partnerschaft von Patent- und Rechtsanwälten mbB
(86) International application number: PCT/KR2016/012672
(87) International publication number: WO 2017/078467

(56) References cited:
- WO-A1-01/62297
- WO-A2-2007/021970
- KR-A- 20080 022 662
- KR-A- 20160 001 419
- US-B2- 8 519 098
- NATSUME ET AL: "Screening of absorption enhancers for nasal peptide and protein delivery", PROCEEDINGS OF THE INTERNATIONAL SYMPOSIUM ON CONTROLLED RELEASE BIOACTIVE MATERIALS, CONTROLLED RELEASE SOCIETY, INC, US; KR, vol. 23, 7 July 1996 (1996-07-07), page 481/482, XP002086011, ISSN: 1022-0178
- NASROLLAHI, S. A. ET AL.: 'Cell-penetrating Peptides as a Novel Transdermal Drug Delivery System' CHEM. BIOL. DRUG DES. vol. 80, no. 5, 2012, pages 639 - 646, XP055083780

## Description

### Technical Field

The present invention relates to a composition for skin penetration, which comprises a cationic molecular carrier and a protein and which aims to deliver the protein into the skin, particularly into a part of the epidermis and the dermis.

### Background Art

Cells are the basic structural unit of all living organisms and are composed of the cytoplasm in which intracellular organelles are present and the cell membrane that protects the cytoplasm and also separates it from the extracellular environment. Since the cell membrane that has a selective permeability of substances has proteins in a fluid state distributed in the phospholipid bilayer in a mosaic pattern, there are many restrictions on the penetration of substances with a useful therapeutic activity through the cell membrane. Particularly, since it is difficult for hydrophilic molecules, highly charged molecules of low molecular weights, and macromolecules such as peptides and oligonucleotides (e.g., nucleic acids and genes) to pass through the cell membrane, several methods for delivering these molecules into cells have been developed *(see* S. Futaki, Adv. Drug Delivery Rev. 2005, 57, 547-558 and P. A. Wender, et al., Adv. Drug Delivery Rev. 2008, 60, 452-472). In reality, however, there have been raised many problems such as causing cytotoxicity.

The cell membrane is one of many biomembranes. Among the various types of biomembranes, examples of biomembranes for which the difficulty of penetration is well known include the blood-brain barrier, the skin/stratum corneum barrier, and the like. It is also well known that certain substances cannot readily pass through the nuclear membrane and the mitochondrial membrane even though they can readily pass through the cell membrane. Furthermore, not all substances that pass through the cell membrane can readily penetrate into various cell organelles surrounded by other biological membranes. Given the nature of the different biomembranes and the difference in permeability between them, it cannot be predicted that a substance that passes through the *in vivo* cell membrane will pass through the skin barrier as well.

The skin is responsible for protecting the body from external stimuli or chemicals, so that it is very difficult for most substances to pass through the skin. It is known that a substance with a low molecular weight, while having adequate fatsolubility and water-solubility, can penetrate the skin. It is generally known that in order for a substance to penetrate the skin, it should have a molecular weight of about 500 Da or less regardless of the type of molecule *(see* Testa et al., Comp. Med. Chem. (2007) p. 292; Naik et al., PSTT Vol. 3, No. 9 Sept. (2000) p. 319).

In the meantime, a variety of molecular carriers that are capable of delivering physiologically active molecules and have a peptide-based structure or a nonpeptide-based structure have been developed *(see* S. K. Chung, et al., Int. J. Pharmaceutics, 2008, 354, 16-22]; K. K. Maiti, et al., Angew. Chem. Int. Ed., 2007, 46, 5880-5884; K. K. Maiti, et al., Angew. Chem. Int. Ed., 2006, 45, 2907-2912; and Korean Patent Nos. 0578732, 0699279, 0849033, and 1021078).

Molecular carriers of these various structures have been applied to deliver various small molecules or such macromolecules as proteins into cells or into skin tissue. For example, covalent bonding, encapsulation with a nanodevice such as a liposome, and complex interactions between protein and protein have been used for the connection between a molecular carrier and a cargo *(see* S. A. Nasrollahi, et. al., Chem. Biol. Drug. Des. 2012, 80, 639-646; W. Shi and S. F. Dowdy, in Cell Penetrating Peptides, Ed. U. Langel, 2007, p201-217; and Y. Hou, et al., Exper. Dermatol. 2007, 16, 999-1006).

WO 01/62297 A1 discloses a method for delivery of a compound to the surface of, into or across a biological barrier, the method comprising contacting the barrier with a composition comprising the compound and a delivery-enhancing transporter, wherein the delivery-enhancing transporter comprises sufficient guanidino or amidino moieties to increase delivery of the compound into or across the barrier compared to delivery of the compound in the absence of the delivery-enhancing transporter.

Natsume et al: "Screening of absorption enhancers for nasal peptide and protein delivery", PROCEEDINGS OF THE INTERNATIONAL SYMPOSIUM ON CONTROLLED RELEASE BIOACTIVE MATERIALS, CONTROLLED RELEASE SOCIETY, INC, US; KR, 7 July 1996 (1996-07-07), page 481/482 discloses chemical enhancers which have a reversible effect and a weak damage to nasal mucosa.However, there has not yet been a successful delivery of a protein into the skin in the form of an ionic complex in which the molecular carrier and the protein form a simple ionic interaction between them.

### Disclosure of the Invention

### Technical Problem

The present inventors have prepared an ionic complex by ionic bonding a molecular carrier previously reported with a protein to a predetermined ratio in order to deliver the protein into the skin and confirmed that the ionically complex thus prepared can improve the skin permeability of the protein, thereby completing the present invention.

Accordingly, an object of the present invention is to provide a composition for skin penetration for the purpose of delivering a protein into the skin.

### Solution to the Problem

In order to achieve the object of the present invention, there is provided a composition for skin penetration, which comprises an ionic complex in which a cationic compound of following Formula 4 and a protein are ionically bonded and which is for delivering the protein into the skin: 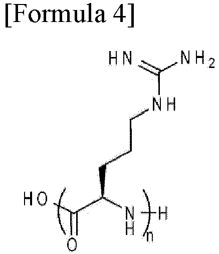 wherein n is an integer of 1 to 8.

### Advantageous Effects of the Invention

The composition according to the present invention exhibits an excellent effect of penetrating proteins having difficulty in passing through the cell membrane or the skin layers into the skin. Thus, it may be effectively used for delivering proteins into the skin.

### Brief Description of the Drawings

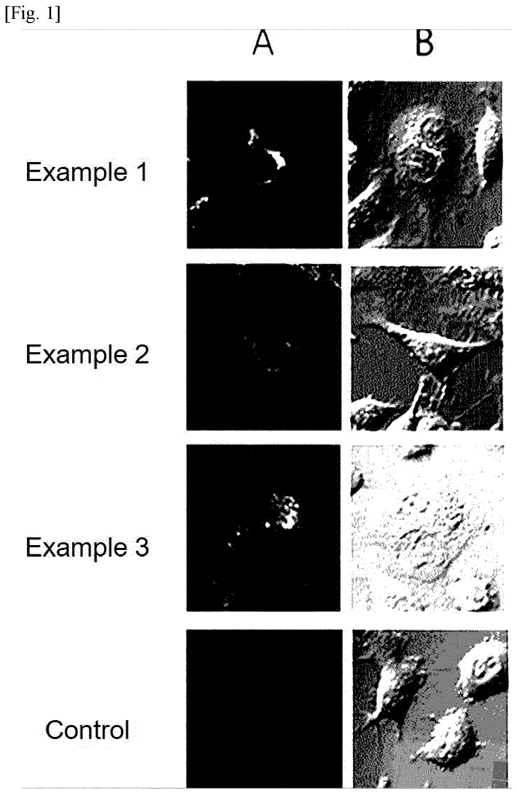

Fig. 1 shows fluorescence images (A) and cell morphological images (B) showing the cell membrane permeability of an ionic complex prepared using the green fluorescent protein according to an embodiment of the present invention.

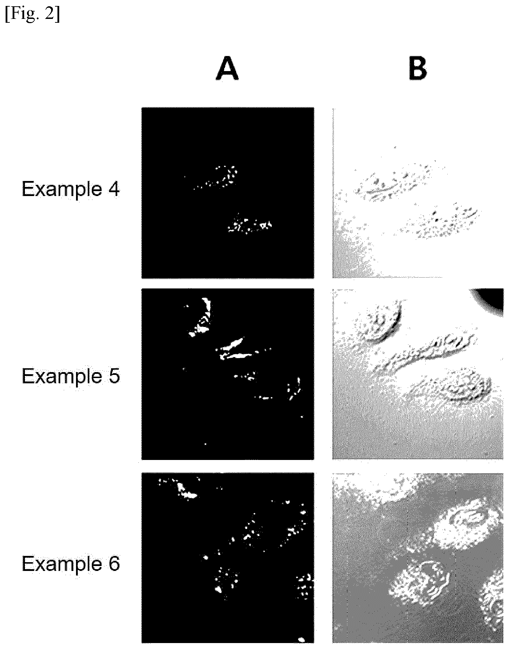

Fig. 2 shows fluorescence images (A) and cell morphological images (B) showing the cell membrane permeability of an ionic complex prepared using albumin-FITC (Example 4), concanavalin A-FITC (Example 5), and immunoglobulin G-FITC (Example 6), respectively, as a protein according to an embodiment of the present invention.

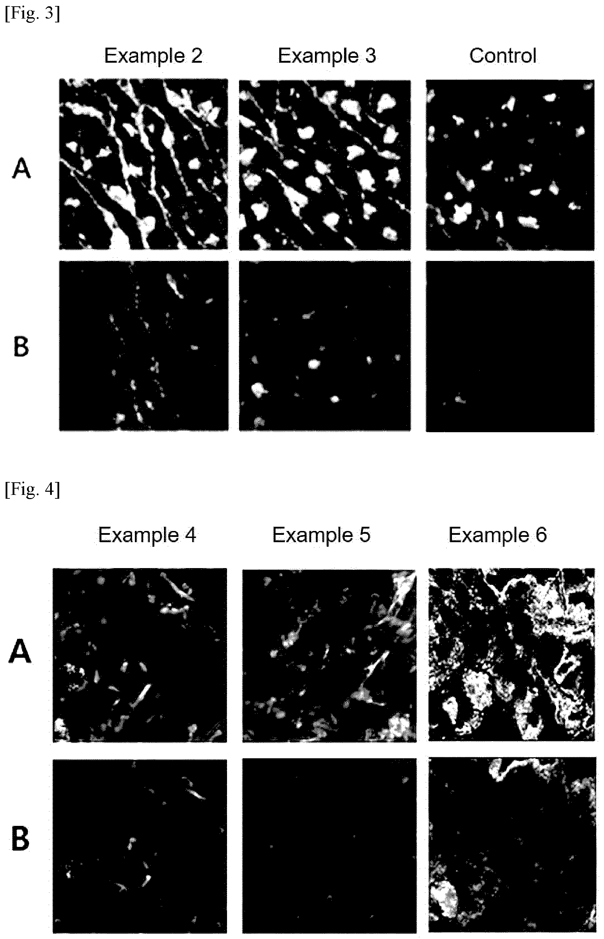

Fig. 3 shows fluorescence photographs of the mouse skin observed at a depth of 33 µm (A) and 63 µm (B), respectively, from the skin surface of a mouse upon the application of an ionic complex prepared using the green fluorescent protein according to an embodiment of the present invention to the skin of the hind leg femur of the mouse.
Fig. 4 shows fluorescence photographs of the mouse skin observed at a depth of 33 µm (A) and 63 µm (B), respectively, from the skin surface of a mouse upon the application of an ionic complex prepared using albumin-FITC (Example 4), concanavalin A-FITC (Example 5), and immunoglobulin G-FITC (Example 6), respectively, as a protein according to an embodiment of the present invention to the skin of the hind leg femur of the mouse.

### Best Embodiment for Carrying Out the Invention

Hereinafter, the present invention will be described in more detail.

The present invention provides a composition for skin penetration, which comprises an ionic complex in which a cationic compound represented by the following Formula 4 and a protein are ionically bonded and which is for delivering the protein into the skin: 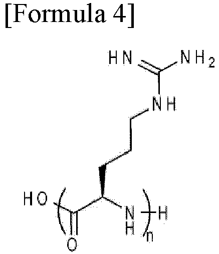 wherein n is an integer of 1 to 8.

In the present invention, the ionic complex comprises a cationic compound. The cationic compound may be any one represented by the above Formula 4 and is used as a molecular carrier *(see,* e.g., Korean Patent No. 0578732, No. 0699279, No. 0849033, and No. 1021078). The cationic compound has a structure in which guanidine groups or arginine groups having a variety of side chain lengths are introduced in a linear or branched form into the sugar or sugar analogue backbone as shown in Formula 4. Since the cationic compound is water-soluble and excellent in the biomembrane permeability, it can readily pass through the cell membrane and the skin layer in the form of an ionic complex bonded with an anionic protein.

The compound according to Formula 4 may be arginine (in case of n being 1) or an arginine oligomer (in case of n being any one of 2 to 8). Specifically, it may be an arginine oligomer wherein n is 6 or 8.

There are no special limitations to the protein comprised in the ionic complex of the present invention as long as it is anionic and can be bonded with the cationic compound to form an ionic complex. Examples thereof include hormones, enzymes, enzyme inhibitors, antibodies, cytokines, fluorescent material, and the like.

Especially, although it is common knowledge in the art that a certain substance should have a molecular weight of about 500 Da or less in order for the substance to penetrate through the skin layer and reach the dermis, the composition for skin penetration according to the present invention has an advantage in that a protein can be efficiently delivered into the skin by forming an ionic complex with the cationic compound as described above even in the case where the protein has a molecular weight far exceeding 500 Da.

For example, the weight average molecular weight of the protein may be 200 kDa or less, 180 kDa or less, 150 kDa or less, 115 kDa or less, 100 kDa or less, 80 kDa or less, 65 kDa or less, 27 kDa or less, or 10 kDa or less.

Specific examples of the protein include green fluorescent protein, red fluorescent protein, epidermal growth factor (EGF), fibroblast growth factor (FGF), antibodies (including a therapeutic monoclonal antibody), lectins, insulin, growth hormone, interferons, interleukins, parathyroid hormone, albumins, streptavidin, concanavalin, immunoglobulin, and the like.

In the ionic complex of the present invention, the cationic compound and the protein may be ionically bonded in a ratio of 1:1 to 20:1, 2:1 to 18:1, 4:1 to 16:1, 5:1 to 15:1, 8:1 to 13:1, or 7.5:1 to 10:1, based on the charge.

In the ionic complex of the present invention, the cationic compound and the protein may be ionically bonded in a ratio of 10:1 to 50:1, 20:1 to 40:1, 12:1 to 30:1, or 15:1 to 25:1, based on the molar ratio.

If the bonding ratio of the cationic molecular carrier and the protein is within the above range in the preparation of the ionic complex, the number of guanidine groups per molecule of the ionic complex is appropriate, so that the cell permeability is improved, and it is possible to prevent the phenomenon that the surplus molecular carriers in a free form, which are not involved in the formation of the ionic complex, penetrate the cell membrane competitively. As a result, it is possible to increase the rate of penetration of the ionic complex to the cell membrane and to improve the efficiency of delivering the protein (or substrate).

The composition for skin penetration according to the present invention penetrates into the skin up to a depth of 50 to 100 µm, 55 to 80 µm, or 60 to 65 µm. Therefore, the composition for skin penetration according to the present invention can penetrate into the cell or under the skin, e.g., between the epidermis and the dermis, to deliver a protein *(see* Test Example 2 and Figs. 3 and 4). Thus, it can greatly improve the delivery of functional cosmetics and therapeutic agents for skin disorders that contain a protein. In addition, it can be advantageously used for delivering a variety of therapeutic or diagnostic agents comprising a protein, which must be subcutaneously administered.

The following example 3 illustrates the invention. Examples 1, 2, 4, 5 and 6 are not part of the invention.

### Example 1: Preparation of an ionic complex comprising a cationic compound of a sorbitol backbone with 8 guanidine groups and the green fluorescent protein (GFP)

The cationic compound (hereinafter referred to as "sorbitol-based G8 molecular carrier" or "SG8") having a sorbitol backbone and 8 guanidine groups (+8; one positive charge per guanidine group) as represented by Formula 2 of the present invention was prepared according to the method described in Example 1 of Korean Patent No. 0699279. Next, 3.5 µg (2.45 nmol) of the SG8 prepared above was dissolved in 16.76 µl of triple distilled water to prepare a solution containing the cationic SG8.

In the meantime, 13.8 µg (0.68 nmol) of the GFP (active A.victoria GFP - Ab84191, 27 kDa, Abcam, -8; 8 negative charges per GFP) was dissolved in 6.76 µl of triple distilled water to prepare a solution containing the anionic GFP.

The solution containing the anionic GFP prepared above was slowly added dropwise to the solution containing the cationic SG8 and mixed, followed by stirring at 0°C for about 30 minutes until the turbid mixed solution became clear. Then, the mixed solution was stored at -20°C for freezing to obtain an ionic complex in which the cationic compound and the GFP were ionically bonded in a charge ratio of 10:1.

### Example 2: Preparation of an ionic complex comprising a cationic compound of a sorbitol backbone with 6 guanidine groups and the GFP

The cationic compound (hereinafter referred to as "sorbitol-based G6 molecular carrier" or "SG6") having a sorbitol backbone and 6 guanidine groups (+6; one positive charge per guanidine group) as represented by Formula 3 of the present invention was prepared according to the method described in Example 8 of Korean Patent No. 0699279. Next, 2.7 µg (2.45 nmol) of the SG6 prepared above was dissolved in 16.76 µl of triple distilled water to prepare a solution containing the cationic SG6.

In the meantime, 13.8 µg (0.68 nmol) of the GFP (-8; 8 negative charges per GFP) was dissolved in 6.76 µl of triple distilled water to prepare a solution containing the anionic GFP.

The solution containing the anionic GFP prepared above was slowly added dropwise to the solution containing the cationic SG6 and mixed, followed by stirring at 0°C for about 30 minutes until the turbid mixed solution became clear. Then, the mixed solution was stored at -20°C for freezing to obtain an ionic complex in which the cationic compound and the GFP were ionically bonded in a charge ratio of 7.5:1.

### Example 3: Preparation of an ionic complex comprising an arginine octamer with 8 guanidine groups and the GFP

The cationic compound (hereinafter referred to as "ARG8") of an arginine octamer (n=8; +8) as represented by Formula 4 of the present invention was supplied by Peptron Inc. Next, 3.11 µg (2.45 nmol) of the ARG8 was dissolved in 16.76 µl of triple distilled water to prepare a solution containing the cationic ARG8.

In the meantime, 13.8 µg (0.68 nmol) of the GFP (-8; 8 negative charges per GFP) was dissolved in 6.76 µl of triple distilled water to prepare a solution containing the anionic GFP.

The solution containing the anionic GFP prepared above was slowly added dropwise to the solution containing the cationic ARG8 and mixed, followed by stirring at 0°C for about 30 minutes until the turbid mixed solution became clear. Then, the mixed solution was stored at -20°C for freezing to obtain an ionic complex in which the cationic compound and the GFP were ionically bonded in a charge ratio of 10:1.

### Example 4: Preparation of an ionic complex comprising a cationic compound of a sorbitol backbone with 6 guanidine groups and fluorescently labeled albumin

29.5 µg (26.6 nmol) of the cationic compound SG6 obtained according to the method described in Example 2 was dissolved in 20.0 µl of triple distilled water to prepare a solution containing the cationic SG6.

In the meantime, 88 µg (0.68 nmol) of albumin-FITC (molecular weight of 66 kDa) was dissolved in 10.0 µl of triple distilled water to prepare a solution containing anionic albumin-FITC.

The solution containing anionic albumin-FITC prepared above was slowly added dropwise to the solution containing the cationic SG6 and mixed, followed by stirring at 0°C for about 30 minutes until the turbid mixed solution became clear. Then, the mixed solution was stored at -20°C for freezing to obtain an ionic complex in which the cationic compound and fluorescently labeled albumin were ionically bonded in a molar ratio of about 40:1.

### Example 5: Preparation of an ionic complex comprising a cationic compound of a sorbitol backbone with 6 guanidine groups and fluorescently labeled concanavalin

29.5 µg (26.6 nmol) of the cationic compound SG6 obtained according to the method described in Example 2 was dissolved in 20.0 µl of triple distilled water to prepare a solution containing the cationic SG6.

In the meantime, 135 µg (0.68 nmol) of concanavalin A-FITC (molecular weight of 102 kDa) was dissolved in 10.0 µl of triple distilled water to prepare a solution containing anionic concanavalin A-FITC.

The solution containing anionic concanavalin A-FITC prepared above was slowly added dropwise to the solution containing the cationic SG6 and mixed, followed by stirring at 0°C for about 30 minutes until the turbid mixed solution became clear. Then, the mixed solution was stored at -20°C for freezing to obtain an ionic complex in which the cationic compound and fluorescently labeled concanavalin A were ionically bonded in a molar ratio of about 40:1.

### Example 6: Preparation of an ionic complex comprising a cationic compound of a sorbitol backbone with 6 guanidine groups and fluorescently labeled immunoglobulin

29.5 µg (26.6 nmol) of the cationic compound SG6 obtained according to the method described in Example 2 was dissolved in 20.0 µl of triple distilled water to prepare a solution containing the cationic SG6.

In the meantime, 199 µg (0.68 nmol) of immunoglobulin G-FITC (molecular weight of 150 kDa) was dissolved in 10.0 µl of triple distilled water to prepare a solution containing anionic immunoglobulin G-FITC.

The solution containing anionic immunoglobulin G-FITC prepared above was slowly added dropwise to the solution containing the cationic SG6 and mixed, followed by stirring at 0°C for about 30 minutes until the turbid mixed solution became clear. Then, the mixed solution was stored at -20°C for freezing to obtain an ionic complex in which the cationic compound and fluorescently labeled immunoglobulin G were ionically bonded in a molar ratio of about 40:1.

### Test Example 1: Measurement of the cell membrane permeability

In order to confirm the cell membrane permeability of the ionic complexes prepared in the above Examples, the fluorescence emitted from the substrate GFP itself was measured with a Confocal Laser Scanning Microscope (Olympus FV1000).

First, HeLa cells (ATCC CCL-2^{™}) were cultured in DMEM (Dulbecco's modified Eagle's medium) containing 10% FBS as a culture medium in a dish plate. Next, a serum-free medium was added to the ionic complexes prepared in Examples 1 to 3 so that the final concentration of the GFP in the ionic complex became 1 µM. The Hela cells were treated with the ionic complex and cultured for 1 hour at 37°C. Thereafter, the cells were washed with PBS 3 times, and the permeability through the cell membrane was immediately observed with a confocal microscope. In the meantime, the GFP only instead of the ionic complex was used for the control group.

An Ar laser (488 nm) was used for the excitation of the GFP, and the cells were observed at a magnification of 40 times. The results are shown in Fig. 1. In Fig. 1, column A shows fluorescence images of the cells treated with the ionic complex, and column B shows morphological images (DIC) of the cells treated with the ionic complex.

As shown in Fig. 1, the cells treated with each ionic complex of Examples 1 to 3 of the present invention showed much stronger fluorescence signals than those of the control group treated with the GFP only.

In addition, the cell membrane permeability of the HeLa cells was observed by confocal microscopy using the ionic complexes prepared in Examples 4 to 6 prepared by the methods as described above, and the results are shown in Fig 2. In Fig. 2, column A shows fluorescence images of the cells treated with the ionic complex, and column B shows morphological images (DIC) of the cells treated with the ionic complex.

As shown in Fig. 2, the cells treated with each ionic complex of Examples 4 to 6 of the present invention showed strong fluorescence signals of the proteins that penetrated into the cells.

### Test Example 2: Measurement of penetration into the mouse skin and distribution therein

In order to confirm the permeability of the ionic complexes prepared in the above Examples into the mouse skin, the fluorescence emitted from the GFP substrate itself was measured with a two-photon laser scanning microscope (Leica).

First, the ionic complexes prepared in Examples 2 and 3 were each diluted with triple distilled water such that the final concentration of the GFP was 24.7 µl. Thereafter, 15 µl of the aqueous solution of the ionic complex was mixed with 50 µl of PEG400 to prepare a sample solution having 15.4% by weight of the GFP.

After 7-week-old BALB/c nude mice were each anesthetized with gas, and 65 µl of the sample solution prepared above was applied to an area of 1 cm × 1 cm on the skin of the hind leg femur. Then, they were left in anesthesia in a dark room for 3 hours. After 3 hours, the samples on the legs were washed with distilled water and 70% ethanol, and the mice were euthanized with carbon dioxide. Subsequently, the femoral skin tissue was peeled off, placed on a glass slide, and fixed with a cover slip. Each slide sample was observed for transdermal permeability with a two-photon laser microscope. A femtosecond laser (wavelength 900 nm) was used to excite the fluorescent material, and the subcutaneous layer was continuously photographed at a depth interval of 3 µm from the skin surface. The images of representative specific depths (33 µm and 63 µm) were observed, and the results are shown in Fig. 3. In Fig. 3, row A shows fluorescence photographs of the mouse skin observed from a depth of 33 µm, and row B shows those observed at a depth of 63 µm.

As shown in Fig. 3, it was confirmed that the ionic complexes of Examples 2 and 3 better penetrate the subcutaneous layer of the mouse as compared with the control group treated with the GFP only. In addition, as the depth of photographing was deepened, the amount of the proteins gradually decreased and the fluorescence intensity weakened. According to the results of transcutaneous penetration measured by a two-photon laser scanning microscopy, it was observed that the proteins with fluorescence penetrated to a depth of at least 63 µm.

Further, the fluorescence emitted from the fluorescently labeled protein as a substrate using the ionic complexes prepared in Examples 4 to 6 as described above was measured with a two-photon laser scanning microscope (Leica). The results are shown in Fig. 4. In Fig. 4, row A shows fluorescence photographs of the mouse skin observed from a depth of 33 µm, and row B shows those observed at a depth of 63 µm.

As shown in Fig. 4, the ionic complexes of the proteins prepared in Examples 4 to 6 well penetrated into the subcutaneous layer of the mouse.

From the above results, it was confirmed that the ionic complexes prepared according to the present invention exhibit a high permeability into the cell membrane and the skin. In addition, it is difficult for proteins having very high molecular weights to be delivered into the subcutaneous layer from the skin surface by a conventional method. However, it was confirmed that such proteins, which are bonded with the cationic compound to thereby form a simple ionic complex according to the present invention, are well delivered into the skin layer and the dermal layer.

Accordingly, the ionic complex according to the present invention can be advantageously used for delivering a protein into a cell or the subcutaneous layer (i.e., the epidermis or the dermis).

## Claims

1. A composition for skin penetration, which comprises an ionic complex in which a cationic compound represented by Formula 4 and a protein are ionically bonded and which is for delivering the protein into the skin: 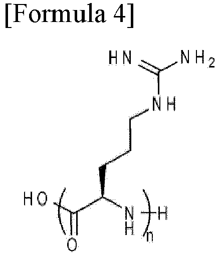 wherein n is an integer of 1 to 8.

2. The composition for skin penetration of claim 1, wherein the cationic compound and the protein are bonded in a ratio of 1:1 to 20:1 based on the charge.

3. The composition for skin penetration of claim 2, wherein the cationic compound and the protein are bonded in a ratio of 7.5:1 to 10:1 based on the charge.

4. The composition for skin penetration of claim 1, wherein the cationic compound and the protein are bonded in a ratio of 10:1 to 50:1 based on the molar ratio.

5. The composition for skin penetration of claim 4, wherein the cationic compound and the protein are bonded in a ratio of 20:1 to 40:1 based on the molar ratio.

6. The composition for skin penetration of claim 1, wherein the protein is any one selected from the group consisting of green fluorescent protein (GFP), red fluorescent protein (RFP), epidermal growth factor (EGF), fibroblast growth factor (FGF), antibodies (including a therapeutic monoclonal antibody), lectins, insulin, growth hormone, interferons, interleukins, parathyroid hormone, albumins, streptavidin, concanavalin, and immunoglobulin.

7. The composition for skin penetration of any one of claims 1 to 5, wherein the weight average molecular weight of the protein is 200 kDa or less.

8. The composition for skin penetration of claim 1, which penetrates into the skin between the epidermis and the dermis.

9. The composition for skin penetration of claim 8, which penetrates into the skin up to a depth of 50 to 100 µm.

## Patentansprüche

1. Zusammensetzung für die Hautpenetration, die einen ionischen Komplex, in dem eine durch Formel 4 dargestellte kationische Verbindung und ein Protein ionisch aneinander gebunden sind, umfasst und die dazu dient, das Protein in die Haut abzugeben: 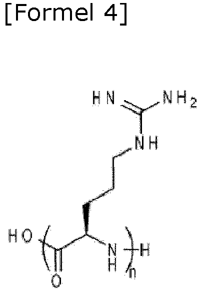 wobei n eine ganze Zahl von 1 bis 8 ist.

2. Zusammensetzung für die Hautpenetration gemäß Anspruch 1, wobei die kationische Verbindung und das Protein in einem Verhältnis von 1:1 bis 20:1 aneinander gebunden sind, bezogen auf die Ladung.

3. Zusammensetzung für die Hautpenetration gemäß Anspruch 2, wobei die kationische Verbindung und das Protein in einem Verhältnis von 7,5:1 bis 10:1 aneinander gebunden sind, bezogen auf die Ladung.

4. Zusammensetzung für die Hautpenetration gemäß Anspruch 1, wobei die kationische Verbindung und das Protein in einem Verhältnis von 10:1 bis 50:1 aneinander gebunden sind, bezogen auf das Stoffmengenverhältnis.

5. Zusammensetzung für die Hautpenetration gemäß Anspruch 4, wobei die kationische Verbindung und das Protein in einem Verhältnis von 20:1 bis 40:1 aneinander gebunden sind, bezogen auf das Stoffmengenverhältnis.

6. Zusammensetzung für die Hautpenetration gemäß Anspruch 1, wobei das Protein beliebig aus der Gruppe ausgewählt ist, die aus dem Grünen Fluoreszierenden Protein (GFP), dem Roten Fluoreszierenden Protein (RFP), dem epidermalen Wachstumsfaktor (EGF), dem Fibroblasten-Wachstumsfaktor (FGF), Antikörpern (einschließlich eines therapeutischen monoklonalen Antikörpers), Lectinen, Insulin, Wachstumshormon, Interferonen, Interleukinen, Parathormon, Albuminen, Streptavidin, Concanavalin und Immunglobulin besteht.

7. Zusammensetzung für die Hautpenetration gemäß einem der Ansprüche 1 bis 5, wobei das Gewichtsmittel des Molekulargewichts des Proteins 200 kDa oder weniger beträgt.

8. Zusammensetzung für die Hautpenetration gemäß Anspruch 1, die zwischen der Epidermis und der Dermis in die Haut eindringt.

9. Zusammensetzung für die Hautpenetration gemäß Anspruch 8, die bis zu einer Tiefe von 50 bis 100 µm in die Haut eindringt.

## Revendications

1. Composition pour pénétrer dans la peau, comprenant un complexe ionique dans lequel un composé cationique représenté par la formule 4 et une protéine sont liés par liaison ionique, et servant pour administrer la protéine dans la peau : 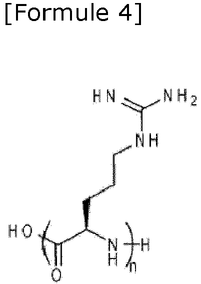 où n est un nombre entier de 1 à 8.

2. Composition pour pénétrer dans la peau selon la revendication 1, dans laquelle ledit composé cationique et ladite protéine sont liés l'un à l'autre dans un rapport de 1 : 1 à 20 : 1, par rapport à la charge.

3. Composition pour pénétrer dans la peau selon la revendication 2, dans laquelle ledit composé cationique et ladite protéine sont liés l'un à l'autre dans un rapport de 7,5 : 1 à 10 : 1, par rapport à la charge.

4. Composition pour pénétrer dans la peau selon la revendication 1, dans laquelle ledit composé cationique et ladite protéine sont liés l'un à l'autre dans un rapport de 10 : 1 à 50 : 1, par rapport au rapport molaire.

5. Composition pour pénétrer dans la peau selon la revendication 4, dans laquelle ledit composé cationique et ladite protéine sont liés l'un à l'autre dans un rapport de 20 : 1 à 40 : 1, par rapport au rapport molaire.

6. Composition pour pénétrer dans la peau selon la revendication 1, dans laquelle ladite protéine est l'une choisie dans le groupe consistant en la protéine fluorescente verte (GFP), la protéine fluorescente rouge (RFP), le facteur de croissance épidermique (EGF), le facteur de croissance des fibroblastes (FGF), des anticorps (incluant un anticorps monoclonal thérapeutique), des lectines, l'insuline, l'hormone de croissance, des des interférons, des interleukines, l'hormone parathyroïdienne, des albumines, la streptavidine, la concanavaline, et une immunoglobuline.

7. Composition pour pénétrer dans la peau selon l'une quelconque des revendications 1 à 5, dans laquelle le poids moléculaire moyen en poids de ladite protéine est 200 kDa ou moins.

8. Composition pour pénétrer dans la peau selon la revendication 1, qui pénètre dans la peau entre l'épiderme et le derme.

9. Composition pour pénétrer dans la peau selon la revendication 8, qui pénètre dans la peau jusqu'à une profondeur de 50 à 100 µm.
